# EUROPEAN PATENT APPLICATION

(11) **EP 4 289 485 A1**
(43) Date of publication of application: **13.12.2023**
(21) Application number: 21923618.9
(22) Date of filing: 23.04.2021
(51) Int. Cl.: A61P 37/04, A61P 43/00, A23K 20/147, A23L 33/17, A61K 38/02

(54) **COMPOSITION HAVING IMMUNOSTIMULATORY EFFECT**

(30) Priority: 04.02.2021 JP 2021016501
(71) Applicant: Asahimatsu Foods Co., Ltd., Iida City, Nagano 399-2561 (JP); Shinshu University, Matsumoto-shi, Nagano 390-8621 (JP)
(72) Inventor: ISHIGURO Takahiro, Iida City, Nagano 3992561 (JP); TANAKA Sachi, Kamiina-gun, Nagano 399-4598 (JP)
(74) Representative: TBK
(86) International application number: PCT/JP2021/016478
(87) International publication number: WO 2022/168341

(57) **Abstract**

Provided is a novel and/or improved means for immunostimulation.

A composition, food and drink, animal feed, or pharmaceutical product containing a resistant protein having an immunostimulatory action. The immunostimulatory action is induction of interferon gamma production by immune cells. The immune cells are spleen cells.

## Description

### Technical Field

The present invention relates to a composition having an immunostimulatory action.

### Background Art

Immunity is a biological defense mechanism that recognizes and eliminates pathogens, toxins, foreign substances, and the like as non-self. In higher animals such as humans, there are advanced systems composed of various proteins (cytokines) and immune cells.

This immune system may decline due to factors such as dietary disorder, aging, and stress.

On the other hand, some food components control the immune system. It has been reported that lactic acid bacteria (Non Patent Literature 1) recognized as so-called probiotics, and a Nozawana insoluble fraction (Non Patent Literature 2) and β-glucan (Non Patent Literature 3), which are prebiotics, have an immunostimulatory action.

It has been reported that the immunostimulatory effect of these food components is an effect via interferon γ. Interferon is a type of cytokine secreted by immune cells in response to foreign substances such as pathogens and tumor cells, and functions to regulate the immune system and inflammation. Interferon γ is produced in activated T cells and NK cells and collects leukocytes at an infected site to enhance inflammation. Interferon γ also has a function of enhancing phagocytosis of macrophages. In general, it is understood that an increase in interferon γ production leads to improvement of immunity.

The Nozawana insoluble fraction and β-glucan are also attracting attention as dietary fibers. Dietary fiber is one of nutritional components recommended to be taken in an amount of 18 to 20 g or more per day. In recent years, various research results have been obtained indicating that dietary fiber acts on intestinal bacteria to produce various health effects.

According to the Codex Alimentarius Commission Committee (Codex Committee) on Nutrition and Foods for Special Dietary Uses (CCNFSDU), dietary fiber is defined as "polysaccharides having a polymerization degree of 10 or more that are not digested and absorbed in the small intestine and are edible polysaccharides originally present in food, polysaccharides obtained by physical, enzymatic, or chemical treatment, and polysaccharides synthesized, and the handling of polysaccharides having a polymerization degree of 3 to 9 is left to the judgment of each country".

It has been found that not only polysaccharides but also other food component residues that have not been digested in the upper gastrointestinal tract contribute deeply to the above-mentioned actions of dietary fiber on intestinal bacteria.

However, the above-mentioned definition of "dietary fiber" by CCNFSDU does not include components other than these polysaccharides.

From such a viewpoint, Association for Dietary Fiber Research proposes a term "luminacoid" as a concept including dietary fibers in a broad sense including components other than polysaccharides. According to the Association for Dietary Fiber Research, luminacoid is defined as "food components that are hard to be digested and absorbed in the small intestine of humans and exhibit physiological effects useful for maintaining health via the digestive tract".

Components included in such a concept of "luminacoid" are roughly classified into non-starch components and starch components. Non-starch components are classified into oligosaccharides, sugar alcohols, resistant proteins, and others in addition to "dietary fibers" in a narrow sense defined by CCNFSDU. On the other hand, starch components are classified into resistant starch and resistant maltodextrin.

Of particular interest among these "luminacoid" components is a "resistant protein" which refers to a protein component or a protein complex component among non-starch components.

As the resistant proteins, soybean-derived proteins such as a soybean high molecular weight protein fraction (HMF) and soybean resistant protein (SRP), proteins derived from buckwheat, proteins derived from silk, proteins derived from sake cake, and the like are known.

From such findings, the resistant protein is expected to be applied to various uses as a food and drink or a pharmaceutical product having a health promoting action.

However, there are still many unclear points regarding the detailed action and effect, and further clarification and enhancement of the action are required for practical use.

### Citation List

### Non Patent Literature

Non Patent Literature 1: Tomomi Ogawa, Teiichi Nishimura, Immunomodulatory action of probiotics, Japanese Journal of Food Microbiology 2013; 30: 1-14
Non Patent Literature 2: Yamamoto K, Furuya K, Yamada K, Takahashi F, Hamajima C, Tanaka S. Enhancement of natural killer activity and IFN-γ production in an IL-12-dependent manner by a Brassica rapa L. Biosci Biotechnol Biochem 2018; 82: 654-668
Non Patent Literature 3: Javmen A, Nemeikaite A, Bratchikov M, Grigiskis S, Grigas F, Jonauskiene I, et al. β-Glucan from Saccharomyces cerevisiae induces IFN-γ production in vivo in BALB/c mice. In vivo 2015; 29: 359-364
Non Patent Literature 4: Naomichi Nishimura, Yuki Mita, Maiko Sakurai, Tatsuo Yamamoto, Toru Ota. Variation analysis of flora in the large intestine by soybean protein indigestible fraction. Soybean protein research 2007; 10: 48-54
Non Patent Literature 5: Norihisa Kato, Nutritional studies of preventive food factors against diseases through functions in the intestine. Journal of Japan Society of Nutrition and Food Sciences 2012; 65: 253-260
Non Patent Literature 6: Lu YC, Yeh WC, Ohashi PS. LPS/TLR4 signal transduction pathway. Cytokine 2008; 42: 145-151

### Summary of Invention

### Technical Problem

One object of the present invention is to provide a novel and/or improved means for immunostimulation.

### Solution to Problem

As a result of intensive studies to elucidate and enhance the action of the resistant protein, the present inventors have found that the resistant protein has an immunostimulatory action of inducing interferon γ production of immune cells, which has not been known so far, and have completed the present invention.

That is, the present invention provides, for example, the following aspects.
[1] A composition containing a resistant protein having an immunostimulatory action.
[2] The composition according to [1], in which the immunostimulatory action is induction of interferon gamma production by immune cells.
[3] The composition according to [1] or [2], in which the immune cells are spleen cells.
[4] A food and drink containing a resistant protein having an immunostimulatory action.
[5] An animal feed containing a resistant protein having an immunostimulatory action.
[6] A pharmaceutical product containing a resistant protein having an immunostimulatory action.

### Advantageous Effects of Invention

According to the present invention, a novel and/or improved means for immunostimulation can be provided. A novel and/or improved immunostimulatory action can be exerted by using a resistant protein.

### Brief Description of Drawings

Fig. 1 is a graph showing supernatant interferon-γ (IFN-γ) concentration after culturing immune cells in the coexistence of a resistant protein and an amino acid mixture constituting the resistant protein.
Fig. 2 is a graph showing supernatant interferon-γ (IFN-γ) concentration after culturing immune cells in the coexistence (PB(+)) and absence (PB(-)) of Polymyxin B for each of a non-added group, a lipopolysaccharide (LPS) added group, and a resistant protein added group.

### Description of Embodiments

Hereinafter, the present invention will be described on the basis of embodiments, but the present invention is not limited to these embodiments, and can be implemented with any modifications within the technical scope understood from the description of the claims.

### [Overview]

The present inventors have conducted various studies on resistant proteins known to have a predetermined lipid metabolism improving effect. As a result, the present inventors have surprisingly found that the resistant protein has an immunostimulatory action which has not been known so far. The present invention is based on such unexpected findings of the present inventors.

That is, one aspect of the present invention relates to a composition containing a resistant protein. This composition is intended for use for immunostimulation.

### [Resistant protein]

In the present invention, the "resistant protein" particularly means, as described above, a protein component or a protein complex component among non-starch components included in the concept of "luminacoid". In the present invention, "luminacoid" is defined as "food components that are hard to be digested and absorbed in the small intestine of humans and exhibit physiological effects useful for maintaining health via the digestive tract" according to the definition of the Association for Dietary Fiber Research described above.

Examples of the resistant protein include proteins derived from various raw materials such as soybean, buckwheat, silk, and sake cake. All of them can be used in the present invention, and among them, soybean-derived proteins are preferable.

Examples of the soybean-derived resistant protein include a protein derived from soybean itself and a protein derived from a soybean processed product, and both of them can be used in the present invention. Examples of the soybean processed product include bean curd, frozen bean curd, soy milk, soybean flour, soybean meat, soybean cereals, soybean protein (SPI), and the like, and any of them may be used. Specific examples of the soybean-derived resistant protein include a soybean high molecular weight protein fraction (HMF), a soybean resistant protein (SRP), and the like, but any of them can be used in the present invention.

As the resistant protein, those isolated and purified from the above-described various raw materials may be used, but the above-described various raw materials including a resistant protein or processed products thereof may be used as they are.

In particular, when the composition of the present invention is used as a food and drink, it is advantageous to use various raw materials containing a resistant protein or most of processed products thereof as they are since they are in the form of a food and drink or a food material.

When the case of a soybean-derived resistant protein is taken as an example, examples of a food and drink or a food material containing the soybean-derived resistant protein include primary processed products such as bean curd, frozen bean curd, soybean milk, soybean protein (SPI), soybean flour, soybean meat, and soybean cereals, secondary processed products such as dairy products, milk substitutes, cereals, processed meats, paste products, and supplements containing the primary processed product, and the like.

As a method for quantifying resistant protein, there is no standardized quantification method like dietary fiber, but it can be quantified by a method conforming thereto. For example, in a case where a sample to be measured is mainly composed of only lipids and proteins and hardly contains other components such as carbohydrates, the sample is subjected to a degreasing treatment, and then sequentially treated with a gastric digestive enzyme and an intestinal digestive enzyme, and the residue after treatment is quantified as a protein component, which can be used as the amount of resistant protein. In addition, in a case where the sample to be measured includes other non-digestible and indigestible components (for example, "dietary fiber" in a narrow sense defined by CCNFSDU) in addition to lipids and proteins, after treatment with a gastric digestive enzyme and an intestinal digestive enzyme by the above-mentioned method, the amount of protein components in the residue is measured by a known protein quantification method (for example, Kjeldahl method, Dumas method, or the like), and this can be used as the amount of resistant protein.

As an example, in the case of quantification of soybean-derived resistant protein, unique quantification methods are known for HMF and SRP, respectively. Any of these may be used, but in particular, the method for quantifying SRP is more preferable as a method for quantifying resistant protein, which is a non-digestible and indigestible protein component, since it is a method actually using a digestive enzyme. The method for quantifying SRP is a method in which a sample is subjected to a degreasing treatment under conditions close to those of digestion in a living body, and then the sample is sequentially treated with pepsin (gastric digestive enzyme) and pancreatin (intestinal digestive enzyme) to obtain a residue as resistant protein. When the sample to be measured is bean curd, separated soybean protein, or the like, components thereof are mainly lipids and proteins, and other components such as carbohydrates are hardly contained. Therefore, the amount of the residue obtained by the enzyme treatment is directly the content of the resistant protein. On the other hand, in the case of a sample also containing other non-digestible and indigestible components, the amount of the protein component in the residue obtained by the enzyme treatment may be measured by a known protein quantification method, and this may be taken as the amount of resistant protein.

Details of the method for quantifying SRP are described in Non Patent Literature 4, but specifically, for example, the method can be performed by the following procedure.

A sample to be measured is subjected to a degreasing treatment, then 3 g of the sample is weighed, and 30 mL of pure water is added thereto to suspend the sample. The pH of this suspension is adjusted to 1.9 with hydrochloric acid, 0.03 g of pepsin is added, and the mixture is reacted at 37°C for 5 hours. Next, the pH of this suspension is adjusted to 7.2 with sodium hydroxide, 0.09 g of pancreatin is added, and the mixture is reacted at 37°C for 14 hours. Thereafter, the enzyme is deactivated by heating at 80°C for 10 minutes, the precipitate remaining after treatment is recovered by centrifugation, and the mass thereof is measured to obtain the amount of resistant protein.

The ratio of the mass of the residue after treatment (that is, the amount of resistant protein) to the mass of the sample before treatment is determined and taken as the content of resistant protein in the sample.

If the sample also contains other non-digestible and indigestible components (such as "dietary fiber" in a narrow sense), the protein component in the residue obtained by the enzyme treatment is quantified by a known method such as Kjeldahl method or Dumas method, and the content of resistant protein in the sample is determined using the quantified protein component.

### [Composition]

The composition of the present invention contains the above-mentioned resistant protein. The content of the resistant protein is not limited, and may be appropriately adjusted according to the use and the like.

The form of the composition of the present invention is not particularly limited, and may be any form such as a solid, a semi-solid, a solution, a suspension, or a dispersion. The form of such a composition can be appropriately selected according to the use of the composition and the like.

The intake amount of the composition of the present invention may be appropriately selected according to the use, type, and species, symptom, age, sex, and the like of the individual taking the composition.

The frequency of intake of the composition of the present invention is not limited, but may be one intake, and according to one example, the composition can be taken, for example, usually at a frequency of 0.5 times or more, or 1 time or more, or 2 times or more per week, and for example, usually at a frequency of 21 times or less, or 14 times or less, or 7 times or less per week. For example, the composition of the present invention can be applied over a period of usually 1 week or more, or 2 weeks or more, or 4 weeks or more. In addition, it may be taken each time, continuously taken, or intermittently taken several times a year at intervals of several months, for example.

### [Use]

The composition of the present invention is used for improving an immunostimulatory action. Interferon γ production is induced by resistant protein, and an immunostimulatory action can be expected. Such findings are novel and unexpected findings that are neither taught nor suggested by conventional findings regarding resistant proteins.

### [Food and drink, animal feed]

Another aspect of the present invention relates to a food and drink and an animal feed containing a resistant protein. One in the form of food and drink is also referred to as "the food and drink of the present invention". Also, among foods and drinks, a food and drink for animals in particular is also referred to as "the animal feed of the present invention".

The food and drink and the animal feed of the present invention are used for improving an immunostimulatory action since interferon γ production is induced by resistant protein and the immunostimulatory action can be expected.

The food and drink of the present invention is not limited, but may be a food with health claims (a food for specified health uses, a food with functional claims, or the like), and in that case, it is preferable that the food and drink is a food with health claims for improving an immunostimulatory action according to various aspects. When the food and drink of the present invention is a feed intended for animals, the use thereof is arbitrary, but it is preferable that the food and drink is a feed intended for improving an immunostimulatory action.

The form and type of the food and drink of the present invention are arbitrary, and are not limited as long as the desired effect of improving an immunostimulatory action by resistant protein is exhibited. Specifically, the form of the food and drink may be any of solid, semi-solid, and liquid (solution, suspension, dispersion, phase separation, or the like). Examples of the food and drink include tea beverages such as green tea, oolong tea, and black tea, coffee beverages, soft beverages, jelly beverages, sports beverages, milk beverages, carbonated beverages, fruit juice beverages, lactic acid bacteria beverages, fermented milk beverages, powdered beverages, cocoa beverages, alcoholic beverages, and purified water; spreads such as butter, jam, furikake, and margarine; mayonnaise, shortening, cream, dressing, bread, rice, noodles, pasta, miso soup, bean curd, milk, yogurt, soup, or sauce; bakery (bread, pies, cakes, cookies, biscuits, crackers, and the like); confectionery (biscuits and cookies, chocolate, candies, cakes, ice cream, chewing gum, tablets, and the like); nutrition supplements (supplements having the form of pills, tablets, jellies or capsules, granola-like cereals, granola-like snake bars, cereal bars), and the like, but any form may be used. Examples of the food and drink include, in addition to normal foods and drinks, various forms such as tablets, chewable tablets, powders, capsules, granules, drinks, gels, syrups, and liquid foods for enteral nutrition.

The food and drink of the present invention may further contain one or more other optional components as long as the desired effect by resistant protein is not substantially disturbed. Examples of such other components include, but are not limited to, active ingredients such as vitamins, minerals, amino acids, and crude drugs, and various pharmaceutical additives such as various food carriers, excipients, surfactants, pH adjusting agents, stabilizers, antioxidants, dyes, flavoring agents, binders, and disintegrants. Any one of these components may be used alone, or any two or more of these components may be used in combination in any combination and ratio.

The content of the active ingredient (resistant protein) in the food and drink of the present invention is not limited as long as the desired effect of immunostimulatory action is exhibited, and may be any amount. The specific active ingredient amount may be appropriately selected according to the component composition, use, type, feeding target, and the like of the food and drink of the present invention.

The production method of the food and drink of the present invention is not limited, but the food and drink can be produced by mixing and cooking the active ingredient (resistant protein) together with one or more other components optionally used, according to a known food production method. The specific production method may be appropriately selected according to the component composition, use, feeding target, type, and the like of the food and drink of the present invention. Alternatively, the food and drink of the present invention can be produced by blending the active ingredient in an existing food and drink.

The details of the animal feed of the present invention are substantially the same as the details of the food and drink of the present invention described above, and can be implemented by appropriately applying various conditions of the food and drink of the present invention described above.

### [Pharmaceutical product]

Another aspect of the present invention relates to a pharmaceutical product containing a resistant protein. One in the form of pharmaceutical product is also referred to as "the pharmaceutical product of the present invention".

The pharmaceutical product of the present invention are used for improving an immunostimulatory action since interferon γ production is induced by resistant protein and the immunostimulatory action can be expected.

The pharmaceutical product of the present invention is preferably, but not limited to, a pharmaceutical product having an immunostimulatory action according to various aspects.

The pharmaceutical product of the present invention may further contain one or more other optional components as long as the desired effect by the active ingredient (resistant protein) is not substantially disturbed. Examples of such other components include, but are not limited to, various pharmaceutically active ingredients, medicinal ingredients such as vitamins, minerals, amino acids, and crude drugs, and further, various pharmaceutically acceptable pharmaceutical additives such as various pharmaceutical carriers, excipients, surfactants, pH adjusting agents, stabilizers, antioxidants, dyes, flavoring agents, binders, and disintegrants. Any one of these components may be used alone, or any two or more of these components may be used in combination in any combination and ratio.

The content of each active ingredient (resistant protein) in the pharmaceutical product of the present invention is not limited as long as the desired effect of immunostimulatory action is exhibited, and may be any amount. The specific active ingredient amount may be appropriately selected according to the component composition, use, administration target, administration route, dosage form, dosage regimen, and the like of the pharmaceutical product of the present invention.

The dosage form of the pharmaceutical product of the present invention is not limited, and examples thereof include oral preparations such as tablets, capsules, granules, powders, syrups, dry syrups, solutions, and suspensions, inhalants, transdermal preparations, enteral preparations such as suppositories, parenteral preparations such as drops and injections, and the like. In the case of a liquid preparation such as a solution or a suspension, the liquid preparation may be dissolved or suspended in water or another appropriate medium immediately before administration. Also, in the case of a tablet, a granule, or the like, the surface thereof may be coated by a known method. Moreover, in the case of an injection, it may be in the form of a solution of sterile distilled water or sterile physiological saline containing a solubilizing agent as necessary. The specific dosage form may be appropriately selected according to the component composition, use, administration target, administration route, dosage regimen, and the like of the pharmaceutical product of the present invention.

The production method of the pharmaceutical product of the present invention is not limited, but the pharmaceutical product can be produced by mixing and formulating the active ingredient (resistant protein) together with one or more other components optionally used, according to a known formulation method. The specific production method of the pharmaceutical product of the present invention may be appropriately selected according to the component composition, use, administration target, administration route, dosage form, dosage regimen, and the like of the pharmaceutical product of the present invention.

The administration route of the pharmaceutical product of the present invention is not limited, but is preferably an administration route in which the desired effect of immunostimulatory action is sufficiently exhibited. Examples include oral administration and enteral administration (tube administration, enteral injection administration, and the like). The specific administration route may be appropriately selected according to the component composition, use, administration target, dosage form, dosage regimen, and the like of the pharmaceutical product of the present invention.

The dosage regimen of the pharmaceutical product of the present invention is not also limited, and an appropriate dosage regimen may be appropriately selected according to the component composition, use, administration target, dosage form, administration route, and the like of the pharmaceutical product of the present invention.

### Examples

Hereinafter, the present invention will be described in more detail with reference to Examples, but these Examples are merely examples shown for convenience of description, and the present invention is not limited to these Examples in any sense.

### [Materials and methods]

### <Preparation of resistant protein>

Frozen bean curd protein was prepared by air-drying a powder obtained by degreasing frozen bean curd powder with a hexane: 2-propanol (3: 2) solvent using a Soxhlet extractor (E-816, Buchi). The frozen bean curd protein was dispersed in about 8 times the amount of water, and the pH was adjusted to 1.9 with hydrochloric acid, then added so that the amount of water was 10 times the amount of protein, and further added with pepsin (Sigma) in an amount corresponding to 1% by mass of the substrate weight. The mixture was reacted at 37°C for 5 hours, then the pH was set to 7.2 with an aqueous solution of sodium hydroxide, and pancreatin (Wako Pure Chemical Industries, Ltd.) was added in an amount corresponding to 3% of the original substrate weight, then the mixture was reacted at 37°C for 14 hours. Thereafter, the enzyme was deactivated by treatment at 80°C for 10 minutes. Next, the pH was adjusted to 4.5 with hydrochloric acid, and the mixture was centrifuged at 3000 × g for 15 minutes to collect a precipitate. The obtained precipitate was repeatedly dispersed in water and centrifuged three times, and a finally obtained precipitate was freeze-dried to obtain a resistant protein.

Hereinafter, the resistant protein thus prepared is referred to as "the resistant protein of this example".

### <Measurement of ability to induce production of interferon γ (IFN-γ) of each material>

Spleen cells were obtained from 6 to 8 week old C57BL/6 mice. Mouse spleen cells were isolated, and its suspension was passed through a 40 µm nylon cell strainer (BD Falcon). Next, cells were treated with 0.17 M Tris-HCl buffer (pH 7.65) containing 0.83% NH4CI to remove red blood cells. The spleen cells were resuspended at a concentration of 10,000,000 cells/mL by adding 10% FBS and RPMI-1640 medium (Sigma) containing penicillin G (100 U/m), streptomycin (100 µg/mL) (Sigma), and 2-Mercaptoethanol. In a 96 well flat bottom plate, spleen cells were seeded at 500,000 cells/well in the coexistence and absence of the resistant protein of this example, an amino acid mixture constituting the resistant protein of this example, a lipopolysaccharide (LPS: Sigma), and Polymyxin B (PB: Sigma, 4 µg/mL), and cultured in a 5% CO2 incubator at 37°C for 48 hours. Thereafter, the IFN-γ concentration of the supernatant was measured by an ELISA kit (eBioscience, San Diego, CA, USA), and used as an index of an ability to induce production of IFN-γ.

### [Results and discussion]

### [Results]

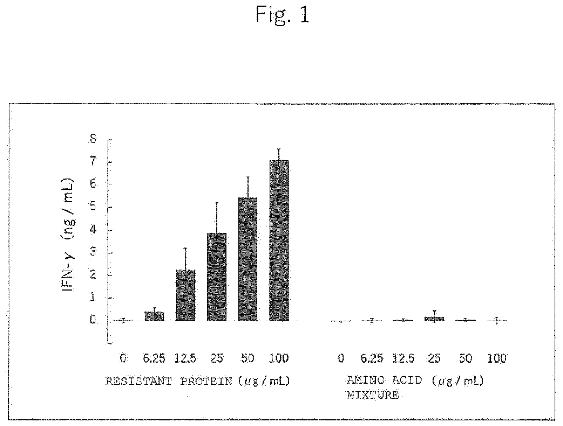

In order to measure the ability to induce production of interferon γ (IFN-γ) by the resistant protein of this example, the resistant protein of this example was added to spleen immune cells and cultured, and then the IFN-γ concentration of the supernatant was measured. As a result, it was shown that the concentration of IFN-γ was increased depending on the concentration of the resistant protein of this example (left in Fig. 1).

Next, when an amino acid mixture in which amino acids constituting the resistant protein of this example were mixed in a free state was examined in the same manner, no ability to induce production of IFN-γ was observed for the amino acid mixture (right in Fig. 1).

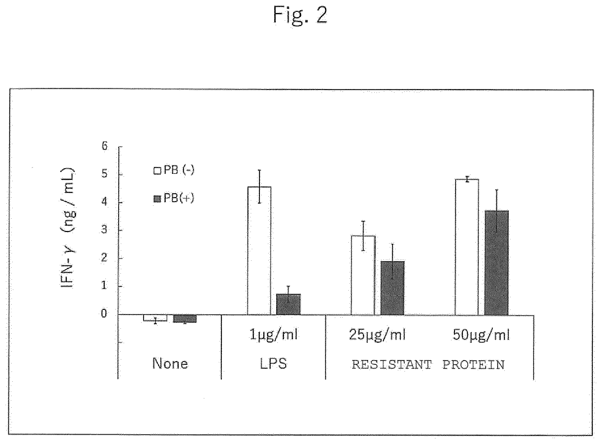

The ability to induce production of IFN-γ by lipopolysaccharide (LPS) was almost suppressed by the presence of Polymyxin B (PB), whereas the ability to induce production of IFN-γ by the resistant protein of this example remained at about 80% even by the addition of PB (Fig. 2).

### [Discussion]

The resistant protein of this example induced the production of interferon γ (INF-γ) of immune cells. From this, it is expected that the composition, food and drink, animal feed, and pharmaceutical product containing the resistant protein of this example have an immunostimulatory action.

As an example in which a relationship between resistant protein and immunostimulation is discussed, there is an influence on intestinal immunity by intake of sericin (silk protein). This is considered to be an effect via intestinal bacterial flora (Non Patent Literature 5).

Therefore, this report is the first example showing a result that the resistant protein directly acted on immune cells.

In order to confirm whether the amino acids constituting the resistant protein as a whole induce production of IFN-γ, the present inventor mixed the amino acids constituting the resistant protein in a free state according to its ratio, and measured the ability to induce production of IFN-γ.

As shown in Fig. 1, since the free amino acid mixture did not have the ability to induce production of IFN-γ, it was considered that the resistant protein of this example had a higher order structure as a protein as one of the keys to exhibit the ability to induce production of IFN-γ.

As in the definition of luminacoid, resistant protein is hardly digested, so that it is considered that the higher order structure of the protein is maintained even in the intestinal tract, and it is considered that the immunostimulatory action is improved by exhibiting the ability to induce production of IFN-γ in immune cells.

Lipopolysaccharide (LPS) is well known as a substance exhibiting ability to induce production of IFN-γ in food components. It is known that the ability to induce production of IFN-γ by LPS is an action via Toll-like receptor 4 (TLR4) (Non Patent Literature 6). Therefore, the ability of LPS to induce production of IFN-γ almost disappeared by the coexistence of Polymyxin B (PB) (Fig. 2).

On the other hand, in the resistant protein of this example, a significant decrease in the ability to induce production of IFN-γ was confirmed by the coexistence of PB, but its decrease rate was smaller than that of LPS, and the ability to induce production of IFN-γ of about 80% remained.

In addition, it has been reported that the ability to induce production of IFN-γ of the Nozawana insoluble fraction is also reduced to 50% or less by the coexistence of PB (Non Patent Literature 2).

From the above, it is considered that improvement of ability to induce production of IFN-γ by the resistant protein of this example is also largely caused by a route other than the route via TLR4, unlike the LPS and the Nozawana insoluble fraction which have been well known so far.

## Claims

1. A composition comprising a resistant protein having an immunostimulatory action.

2. The composition according to claim 1, wherein the immunostimulatory action is induction of interferon gamma production by immune cells.

3. The composition according to claim 1 or 2, wherein the immune cells are spleen cells.

4. A food and drink comprising a resistant protein having an immunostimulatory action.

5. An animal feed comprising a resistant protein having an immunostimulatory action.

6. A pharmaceutical product comprising a resistant protein having an immunostimulatory action.
